(19)

Europäisches
Patentamt
European
Patent Office
Office européen
des brevets

(11)　**EP 4 116 414 A1**

(12)　**EUROPEAN PATENT APPLICATION**

(43) Date of publication:
**11.01.2023　Bulletin 2023/02**

(21) Application number: **21305965.2**

(22) Date of filing: **09.07.2021**

(51) International Patent Classification (IPC):
***C12N 9/02*** *(2006.01)*

(52) Cooperative Patent Classification (CPC):
**C12N 9/0008; C12Y 102/01002**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
- **Centre National de la Recherche Scientifique
  75016 Paris (FR)**
- **Université d'Aix-Marseille
  13007 Marseille (FR)**

(72) Inventors:
- **MAGALON, Axel
  13001 MARSEILLE (FR)**
- **ARIAS-CARTIN, Rodrigo
  83470 ST-MAXIMIN-LA-STE-BAUME (FR)**
- **WALBURGER, Anne
  13008 MARSEILLE (FR)**

(74) Representative: **LLR
  11 boulevard de Sébastopol
  75001 Paris (FR)**

Remarks:
The complete document including Reference
Table(s) and the Sequence Listing(s) can be
downloaded from the EPO website

(54)　**A FORMATE DEHYDROGENASE, ITS USES AND A METHOD FOR CO2 FIXATION**

(57)　The invention relates to an YjgCD-type protein complex, wherein said complex has an enzymatic activity of formate dehydrogenase and/or of carbon dioxide reductase and the complex is oxygen resistant. The complex comprises at least a YjgC-type protein and YjgD-type protein, said YjgC-type protein comprising an amino acid sequence as set forth in SEQ ID NO: 1 or any homologous sequence derived from said sequence SEQ ID NO: 1 and the YjgD-type protein comprising an amino acid sequence as set forth in SEQ ID NO: 3 or any homologous sequence derived from said sequence SEQ ID NO:3.

EP 4 116 414 A1

**Description**

**Field of the invention**

The invention relates to a formate dehydrogenase/ carbon dioxide reductase enzyme and its uses for carbon dioxide fixation and/or formic acid, or formate, production.

**Prior art**

Given the climatic effects linked to the increase in atmospheric $CO_2$, strategies based on the valorisation of $CO_2$ for the production of useful carbon molecules are of social and economic outmost interest. The existing processes usually have a very high energy cost. A "green" approach to $CO_2$ bio-reduction could be a real alternative as living organisms transform $CO_2$ into useful carbon molecules under mild physiological conditions. However, their use for biological synthesis allows yields that are still too low to be of industrial interest (Gleizer, S. *et al.* 2019).

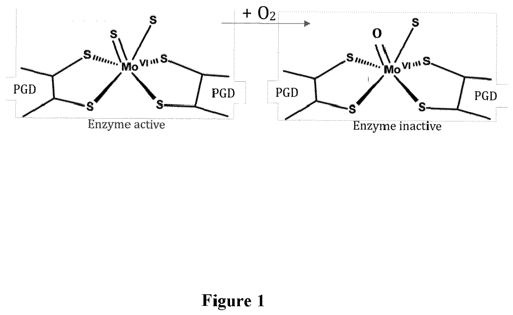

Isolated enzymatic systems can transform $CO_2$, one of the most stable molecules in our atmosphere, by using activation strategies developed over several billion years of evolution (Kumar, B. *et al.* 2012). Formate dehydrogenases (FDHs) are enzymes from the Mo/W-*bis*PGD (molybdenum/tungsten-bis pyranopterin guanine dinucleotide) family which interconvert $CO_2$ and formic acid / formate and to do this, they use active sites based on molybdenum or tungsten, which are abundant transition metals on Earth (Grimaldi, S *et al.* 2013). FDHs are a group of heterogeneous enzymes involved in energy harvesting or $CO_2$ fixation. They display different subunits composition and quaternary structures from single monomeric subunit to the heterotetrameric complex formyl-methanofuran dehydrogenase. In multimeric FDHs, the genes encoding catalytic and partners subunits are organized into an operon. The partner subunits can hold a variety of redox cofactors ([Fe-S] clusters, hemes, Fe-Fe center, $F_{420}$ or FMN) and are the most variable element of the FDHs (Maia et al., 2017; Nielsen et al., 2019; Niks and Hille, 2019). However, these natural catalysts are found in strict anaerobic bacteria and archaea and/or participate in anaerobic metabolic pathways. Their enzymatic activity is therefore negatively impacted by oxygen. The oxygen inactivates the active site of FDHs by replacing the sulfido group in the first coordination sphere of the metal essential for the catalysis by an oxo group (as represented in Figure 1).This sensitivity to oxygen is a major obstacle to their use in biotechnological processes, as is their low catalytic efficiency for the reduction of $CO_2$ into formate (Nielsen *et al.*, 2019). The use of microorganisms such as *Acetobacter iovabiensis* is described in WO2013/011292 in order to fix carbon dioxide and convert it into carboxylic acid.

**Description of the invention**

A new type of FDH has now been identified which presents surprising tolerance to oxygen. The FDH is present in *Bacillus subtilis.* The archetypal gram-positive bacterium, *Bacillus subtilis,* is not able to produce formate (Nakano et al., 1997) although FDH activity was previously detected in cells grown aerobically in rich medium (Glaser et al., 1995). Although *B. subtilis* is a widely characterised bacterium, the FDH of the invention has never been identified, and studied, most likely because it presents atypical characteristics for FDHs. Indeed, it presents an unprecedented structural organization including a novel partner subunit and the immediate environment of its active site is distinct from all the FDHs already characterised. The FDH from *B. subtilis* is composed of two subunits; one is required for the catalytic activity while the unprecedented partner subunit is of unknown function. Finally, the hitherto archetypal conserved residues surrounding the active site of FDHs are substituted by other residues in the FDH of the invention and confer oxygen resistance properties (Figure 1).

Unexpectedly the FDH according to the invention can retain up to 80% of its activity after 14hrs of atmospheric oxygen exposure. This result is particularly remarkable since the kinetics of FDH inactivation by oxygen is very fast. By contrast it takes less than 20 min for the FDH from *Escherichia coli* (Axley, M.J. et al. 1990) to be inactivated by oxygen.

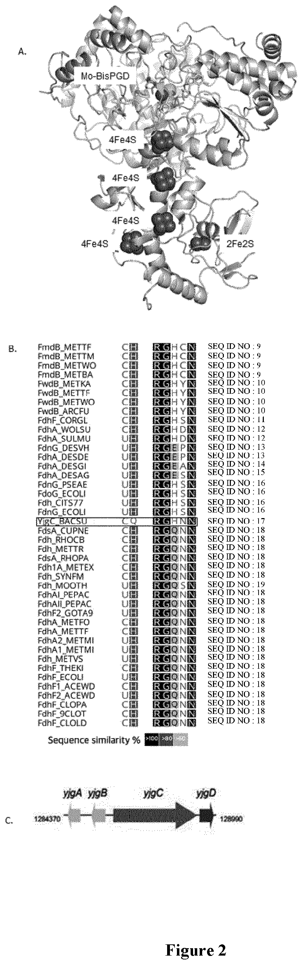

Scrutiny of the *B. subtilis* 168 genome indicates that it encodes for *yjgC* gene, whose ~110 kDa predicted product contains a C-terminal domain typical for Mo/W-bisPGD enzymes and characterized by motifs allowing the coordination of one [4Fe-4S] cluster and one Mo/W-*bis*PGD cofactor (Figure 2A). Interestingly, the Mo-*bis*PGD enzyme sequence contains a N-terminal extension that holds three [4Fe-4S] and one [2Fe-2S] cluster binding motifs and shares homologies with the N-terminal domain of the NuoG subunit of the NADH:ubiquinone oxidoreductase and the catalytic subunit of NAD-dependent [NiFe]-hydrogenases (Zuchan et al., 2021). A close-up alignment of the active site residues of 40 characterized FDH sequences and YjgC sequence is shown in Figure 2B. While the typical metal ligand cysteine residue and the strictly conserved Arg residue are present, the conserved His residue is substituted by a Gln in YjgC. The absence of the His residue immediately to the C-terminal side of the Cys residue questions the activity of this enzyme as FDH. Interestingly, the *yjgC* gene is predicted to be organized into an operon as it is co-expressed with *yjgD* with a positive correlation of 0.98 (Nicolas et al., 2012; Zhu and Stülke, 2018) (Figure 2C). The *yjgD* gene encodes a 21kDa predicted product with the presence of a domain of 38 amino acids of unknown function DUF1641 (PFAM PF07849).

While YjgC is related to FdsA from *Rhodobacter capsulatus,* as illustrated by the YjgC structural model based on the 3D-structure of FdsA (Figure 2A), YjgD sequence is very distinct from the FdsBGD sequences that compose the FDH complex from *R. capsulatus* which couples formate oxidation to NADH reduction (Hartmann and Leimkühler, 2013). YjgD is not predicted to harbor cofactor and is distinct from the sequence of the FdsC sulfurtransferase or any specific chaperone involved in Mo-*bis*PGD maturation (Böhmer et al., 2014; Magalon and Mendel, 2015).

It has now been found a novel FDH complex that shows substantial resistance to the presence of oxygen whilst being able to catalyse the $CO_2$/formate reaction. This FDH complex comprises a novel FDH (an YjgC-type protein) and a novel FDH co-factor (an YjgD-type protein). An object of the invention is therefore a YjgC-type protein, which advantageously comprises an amino acid sequence as set forth in SEQ ID NO: 1 or any homologous sequence derived from said sequence SEQ ID NO:1 by substitution, addition or deletion of at least one amino acid. Preferably, said homologous sequence retains at least 60%, such as at least 65 %, preferably at least 70 %, and even more preferably at least 99 % of identity with the amino acid sequence set forth in SEQ ID NO:1.

Advantageously the amino acid sequence of the YjgC-type protein according to the invention does not have a histidine in position 392 and/or in position 392 of a FDH consensus sequence. It is further preferred that this amino acid in position 392 be a glutamine. The YigD-type protein of the invention preferably acts as co-factor and enhances or allows the enzymatic reaction.

Another object of the invention is YjgD-type protein, said protein comprising an amino acid sequence as set forth in SEQ ID NO: 3 or any homologous sequence derived from said sequence SEQ ID NO:3 by substitution, addition or deletion of at least one amino acid, provided that said homologous sequence retains at least 60 %, such as at least 65 %, preferably at least 70% and advantageously at least 99 % of identity with the amino acid sequence set forth in SEQ ID NO:3. The YigC-type protein of the invention preferably shows an enzymatic activity and/or is the site of the enzymatic reaction.

By "protein", it is meant an amino acid chain containing molecule harbouring a biological function and which naturally occurs in an organism, said biological function being part of a natural process of the organism.

By "at least 60% of identity with the sequence as set forth in SEQ ID NO : X", it is meant 60 %, 61 %, 62 %, 63 %, 64 %, 65 %, 66 %, 67 %, 68%, 68 %, 70 %, 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, 99 % and 100 % of identity with the sequence as set forth in SEQ ID NO : X. Regarding the percentage of identity, it is defined by the percentage of amino acid residues of SEQ ID NO : 1 which align with the same amino acid in the sequence of the homologous protein. The sequence alignment is carried out using dedicated algorithms and programs (such as ClustalW).

In the invention, the term "comprising" is meant to include the terms "consisting essentially of" and "consisting of".

Advantageously, said YigC-type and/or YjgD-type protein or said derived protein originates from a microorganism belonging to the *Bacillales* order, and particularly is

*Bacillus subtilis.*

A further object of the invention is YjgCD-type protein complex, wherein said complex comprises at least an YjgC-type protein and YjgD-type protein as described in this specification. Preferably the complex has an enzymatic activity of formate dehydrogenase and/or of carbon dioxide reductase. The YjgCD-type protein complex of the invention is preferably oxygen resistant or oxygen tolerant, these two adjectives being used conversely. Being oxygen tolerant and/or resistant means that the complex can tolerate relatively high levels of oxygen without damaging the complex or its activity. Alternatively or additionally it may also mean that the complex can maintain its activity through a substantial period of time in presence of oxygen and/or an oxygen containing medium. Preferably the complex can function at an oxygen level of more than 10% (v/v), preferably more than 15 % (v/v) and more preferably of more than, or about, 20% (v/v). Advantageously the enzymatic complex of the invention can function in the presence of air or of a gaseous medium having an oxygen content ranging from 19 to 22 % (v/v). It is further preferred than the enzymatic complex of the invention be able to maintain its enzymatic activity for a period of at least 1 hour, preferably at least 10 hours, e.g. at least 16 and even for at least a day, a week or a month. By "retaining its activity" it is meant inter alia that the quantity of substrate transformed by the enzymatic complex per unit of time y in the absence of oxygen is retain by 60% (v/v) when oxygen is present. Advantageously this rate is more than 65% (v/v) advantageously more than 70% and even more preferably more, or equal to 80% (v/v). In particular the complex of the invention can retain up to 80% of its activity after 14 hours of atmospheric oxygen exposure.

The term complex used in the "expression YjgCD-type protein complex" is not solely descriptive of an association of molecules not connected by covalent bonds. This expression also extends to chimeric molecules which, for example, would associate YjgC-type protein and YjgD-type protein together and connect them in a covalent manner.

The enzymatic activity of the complex of the invention can be formate dehydrogenase. This can be particularly useful for the complete recycling of formic acid in a H2 gas production process. Preferably, the enzymatic activity of the complex according to the invention is a carbon dioxide reductase. This function would be particularly useful for the capture of $CO_2$ as above discussed.

A further object of the invention is a recombinant vector comprising a gene encoding for an YjgC-type protein, an YjgD-type protein and/or an YjgCD-type protein complex of the invention as defined in the specification.

A yet further object of the invention is a recombinant microorganism comprising a gene encoding for a YjgC-type protein , a YjgD-type protein, and/or a YjgCD-type protein complex of the invention and as defined in the specification.

The complex of the invention can be part of a recombinant microorganism which would contain heterologous genes encoding for at least part of the enzyme complex of the invention. It is however preferred that the enzymatic complex of the invention is completely extracellular to a microorganism. This would allow formation of a product such as formic acid to take place outside microorganisms' cells and advantageously outside microorganism.

The enzymatic complex of the invention can comprise one or more additional enzymes or other compounds to increase or improve the catalytic activity of the complex. For example, the following compound could be added to the complex: the menaquinone.

Another object of the invention is a method of synthesis of either $CO_2$ or formate (or formic acid) by using the enzymatic complex of the invention and a substrate This method comprises a step of contacting the enzymatic complex of the invention with a medium containing either formic acid or $CO_2$ as a substrate and providing suitable conditions for the enzymatic reaction to take place and for the production of $CO_2$ or formate, respectively.

Another object of the invention is a method for producing formic acid and/or fixing carbon dioxide, said method comprising the steps of contacting a medium comprising an YjgC-type protein, an YjgD-type protein, an YjgCD-type protein complex, and/or a recombinant microorganism of the invention and as defined in this specification and carbon dioxide. Advantageously suitable conditions for the reaction to take lace are set.

The suitable conditions above mentioned can comprise the use of an aqueous medium where the substrate is dissolved to a given concentration such as 50 mM formate/50 mM $CO_2$.

It can further comprise adjusting the pH, for example to a pH ranging from 9 to 11, preferably from 9.5 to 10.5 and advantageously around $10 \pm 0.2$.

A further object of the invention is the use of the YigC protein as a formate dehydrogenase and/or of carbon dioxide reductase.

Yet a further object of the invention is the use of an YjgC-type protein, an YjgD-type protein, an YjgCD-type protein complex and/or a recombinant microorganism as herein described to carry out the oxidation of formate to $CO_2$ and/or the reduction of $CO_2$ to formate.

**Brief description of the Figures**

Figure 1: Impact of oxygen on the active site chemistry of typical FDH

Figure 2:

2A - Structural model of YjgC generated with i-tasser using FdsA from R. *capsulatus* (PDB ID: 6TGA).
2B - Close up on the alignment of amino acids involved in the active site of 38 known FDH enzymes and the YjgC protein from *B. subtilis*
2C - Genomic organization of *yjgCD* genes in *B. subtilis* encoding the FDH

Figure 3: 3A - picture of SDS-polyacrylamide gel electrophoresis of purified YjgCD; 3B: Michaelis-Menten plot for the reaction of YjgCD with different formate concentrations in presence of benzyl viologen; 3C: shows the pH dependency of catalytic turnover $K_{cat}$ for YjgCD.

Figure 4: shows the activity of YjgCD from *Bacillus subtilis* when exposed, or not, to air.

Figure 5 is the amino acid sequence SEQ ID NO: 1 which corresponds to the protein YjgC (UniProt database) of *Bacillus subtilis* 168, ref | BSU_12160 | of UniProt database.

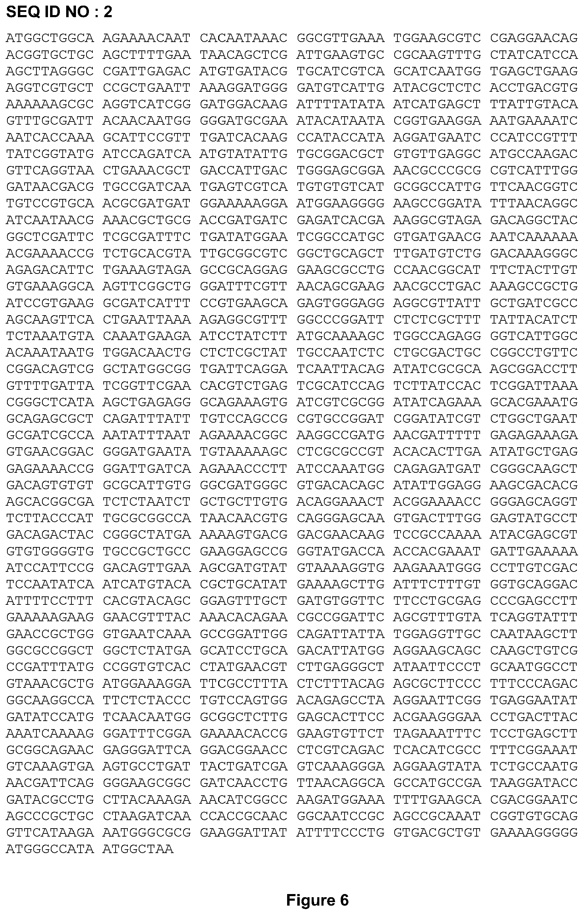

Figure 6: is the nucleotide sequence SEQ ID NO: 2 which corresponds to the gene *yjgC* of *Bacillus subtilis* 168, ref. | BSU_12160 | of UniProt database.

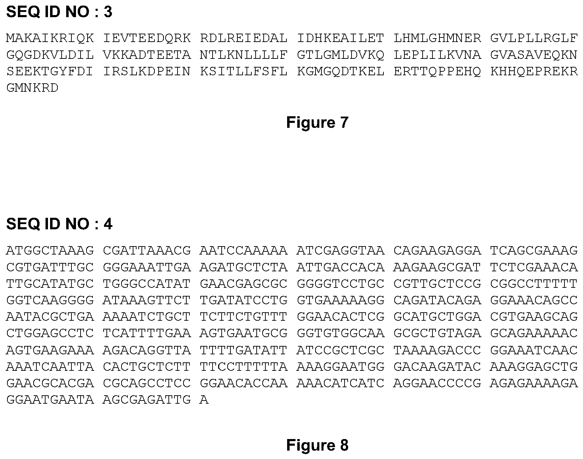

Figure 7: is the amino acid sequence SEQ ID NO: 3 which corresponds to the protein YjgD of *Bacillus subtilis* 168, ref. | BSU_12170 | of UniProt database.

Figure 8: is the nucleotide sequence SEQ ID NO: 4 which corresponds to the gene *yjgD* of *Bacillus subtilis* 168, ref. | BSU_12170 | of UniProt database.

## Methods

### Bacterial strains, media, and culture conditions

Genomic integration of reporter sequences to regulatory sequence of *yigC* and *yigD,* shown in Figure 2A, was achieved using the pSPH1 plasmid by transformation at the *amyE* locus (Dubnau et *al.*, 1971). Target integration was confirmed by amylase sensitivity. Clones were verified by PCR. Strains used are described in table 3.

For cloning the pET28HT-YjgCD plasmid, *yjgCD* was amplified with primers 898-899 and introduced into pET28dHT-*gapR* (Arias-Cartin et al., 2017) at *Nde*I-*Kpn*I sites. This adds in 5' end of *yjgC* a sequence encoding a 6-his tag followed by a TEV cleavage site. Then *yjgCD* was amplified with primers 941-952 using pET28HT-YjgCD and introduced into pSHP1 at *Avr*II-*Xho*I sites to give the pSPH1-6his*yjg*CD plasmid.

The *yjgCD* operon is under the control of a xylose inducible promotor. The resulting plasmid was verified by sequencing. Plasmids and primers are described in Tables 1 & 2 below:

Table 1: Primers

| Primer | Sequence (5' to 3') |
| --- | --- |
| 898 | atttgtatttccaaggtcatatggctggcaagaaaacaatcacaa |
| 899 | tcgagtgcggccgcaagcttcaatctcgcttattcattcctctt |
| 941 | atgcatctagaaaggagattcctaggatggctcaccaccaccaccaccac |
| 952 | caagcttatcgataccgtcgacctcgagtcaatctcgcttattcattcc |

Table 2: Plasmids

| Plasmid | Number | Description |
| --- | --- | --- |
| pET28HT-YjgCD | 4084 | *yjgCD* was amplified with primers 898-899 and introduced into pET28dHT-*gapR* (Arias-Cartin et al., 2017) at *Nde*I-*Kpn*I sites. This adds in 5' end of *yjgC* a sequence encoding a 6-his tag followed by a TEV cleavage site. |
| pSPH1-6his*yjg*CD | 4085 | *yjgCD* was amplified with primers 941-952 using 4084 and introduced into pSHP1 at *Avr*II-*Xho*I sites. |

Table 3: Strains used

| Strains | Description | Reference |
| --- | --- | --- |
| 4161 | *Bacillus subtilis 168 trpC* | BGSC |
| 4088 | *Bacillus subtilis 168 trpC2 amyE::pSHP1-6hisyjgCD-plasmid 4085 was transformed into 4161* | |

For routine growth, cells were propagated in a Lysogenic Broth (LB) medium. When necessary, antibiotic was used at the following concentrations: spectinomycin (100 $\mu$g/mL).

*B. subtilis* 168 derivative (4088) was grown in 4.8 L of LB medium with 2 $\mu$M sodium molybdate at 37°C until optical density (OD) reached 0.3. Cells were then submitted to a salt shock (0,4 M NaCl) during 1 hours to induce SigB-dependent general stress genes which could be involved in YjgC folding and maturation as the biogenesis of molybdenum-containing enzymes is a very complex process (Magalon and Mendel, 2015). Expression of recombinant proteins was induced with 0.8% xylose during 3 hours. Harvested cells were washed once in a buffer containing 20 mM sodium phosphate pH 7.5 and 50 mM $Na_2SO_4$ and cells were kept at -80°C.

### Purification of 6his-YjgCD

All steps were performed at 4°C with an Akta FPLC system (GE Healthcare). Frozen cells, typically 10-15 g of wet weight, were thawed, suspended in 90 mL of 20 mM sodium phosphate pH 7.5, 50 mM $Na_2SO_4$. Cell suspension was treated with proteases inhibitor cocktail (Roche), lysozyme (1 mg/mL-30 min at 37°C), DNAse (powder-20 min at RT) and EDTA 0.5 mM and disrupted by 1-2 passages through a French pressure cell at 1 bar. Cell debris were removed by centrifugation at 100,000 x g during 40 min. The supernatant was filtered, 50 mM imidazole was added and it was loaded onto 3 x 5 mM Ni-NTA affinity columns (GE healthcare). Columns were washed with 20 mM sodium phosphate pH 7.5 50 mM $Na_2SO_4$ 50 mM imidazole and the enzyme was eluted in 20 mM sodium phosphate pH 7.5 50 mM $Na_2SO_4$ 500 mM imidazole and 10 mM $KNO_3$ was added. Enzyme was washed in 50 mM MES pH 6, 50 mM $Na_2SO_4$, 5% Glycerol, 10 mM $KNO_3$ with PD-10 columns (Pharmacia), concentrated with Amicon 100 kDa (Millipore) and frozen in liquid nitrogen.

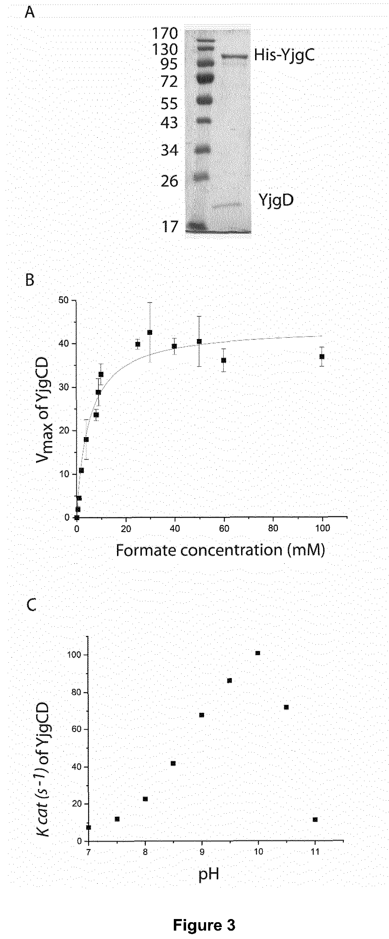

Purified proteins were analysed using Coomassie Brilliant Blue-stained 12% SDS-polyacrylamide gel, Figure 3A. Molecular standards are indicated in kDa. YjgC 6his-tagged in N-terminal (His-YjgC) was overproduced in *B. subtilis* 4088. It co-purifies with the YjgD protein which displays a band with a predicted monomeric mass of 21.3 kDa.

*Activity Assays and Kinetic Analysis*

FDH activity was determined using an Avaspec-2048L spectrometer (Avantes), inside an anaerobic glovebox filled with an atmosphere of 100% $N_2$. The experiments were carried out at room temperature (25°C) with stirring. The reaction of YjgCD with different formate concentrations was carried out in presence of benzyl viologen in 25 mM CHES, pH 10 and the analysis is shown as a Michaelis-Menten plot in Figure 3B.

$$\text{Michaelis-Menten equation: } y = V_{max} \times x/(K_M + x)$$

For formate::benzyl viologen OR measurements, the reduction of 17.5 mM benzyl viologen (BV) was monitored at 600 nm ($\varepsilon_{600\,nm}$ $BV^+$ = 7.0945 $mM^{-1}$ $cm^{-1}$) in 25 mM CHES pH 10 after addition of 50 mM sodium formate. One unit of oxidizing activity is defined as the amount of FDH capable of oxidizing 1 $\mu$mol of formate par min per mg of enzyme.

The influence of pH on FDH activity was assessed using a buffer mix containing 20 mM MES, CHAPS, CHES, HEPES. The pH was adjusted from 6.5 to 11 with HCl and NaOH. The pH dependency of $K_{cat}$ for YjgCD is shown in Figure 3C. The analysis was carried out at room temperature (25°C).

*Oxygen tolerance*

The purified YjgCD in solution into 50 mM MES pH 6, 50 mM $Na_2SO_4$, 5% Glycerol was exposed to air during 1, 2, 3, 4 and 15 hours at RT. The remaining activities were measured periodically during the air oxidation procedure. To remove dissolved $O_2$ from the enzyme solution, the samples were flushed with $N_2$ and diluted 800 times in anaerobic buffer. All assays were performed under the same buffer conditions of 25 mM CHES buffer at pH 10 and at room temperature. The activity of YjgCD kept in anoxic conditions into 50 mM MES pH 6, 50 mM $Na_2SO_4$, 5% Glycerol during 1, 2, 3, 4 and 15hrs at room temperature is identical to the activity at time 0 (100%=28,1U/mg of YjgCD).

**1. The YjgCD is a protein complex with formate oxidizing activity**

To demonstrate the activity of YjgC as FDH and the importance of YjgD in this activity, corresponding proteins were purified in *B. subtilis.* First the *yjgCD* operon was expressed under the control of an inducible promoter and the YjgC protein to which a 6-histidine tag was added at the N-terminus was purified. Affinity purification of these proteins showed that YjgC was copurified with YjgD as identified by SDS-PAGE and mass spectrometry analysis (Figure 3A). A full steady-state enzyme kinetic analysis of formate oxidation by YjgCD using benzyl viologen as artificial electron acceptor is shown in Figure 3B. Using the Michaelis-Menten plot, we have obtained for the YjgCD heterodimer (MW: 132 kDa) a $K_{cat}$ of 96 $s^{-1}$ (Table 4). Consistent with previous reports (Axley et al., 1990; Hartmann and Leimkühler, 2013; Schuchmann and Müller, 2013), enzyme preparation exhibit a basic pH optimum, pH 10 for the YjgCD (Figure 3C). In conclusion, the YjgCD is a protein complex displaying formate oxidizing activity.

Table 4: Enzymatic catalytic constants for Formate oxidation by YjgCD

| FDH | $K_M$ formate (mM) | $K_{cat}$ ($s^{-1}$) | $V_{max}$ (U) |
|---|---|---|---|
| YjgCD | 5,1 $\pm$ 0,9 | 96,1 $\pm$ 4 | 43,7 $\pm$ 1,9 |

**2. YjgCD is resistant to air and/or O2 exposure**

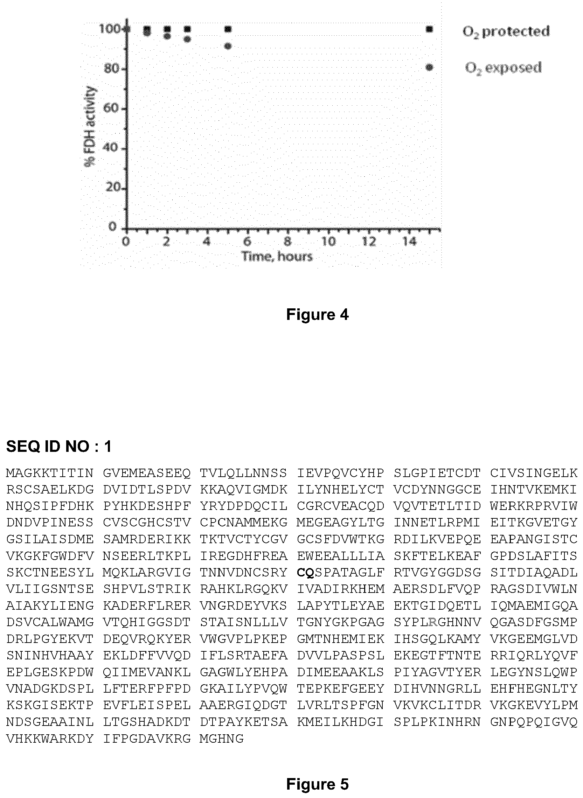

The purified YjgCD was exposed to air during 1, 2, 3, 4 and 15 hours at room temperature (25°C). Unexpectedly, YjgCD can retain up to 80% of its activity after 14 hours of atmospheric oxygen exposure (Figure 4). This result is particularly remarkable since the kinetics of known FDH inactivation by oxygen is very fast. For example, it takes less than 20 min for the FDH from *Escherichia coli* to be inactivated by oxygen. Moreover, this ability to substantially resist oxygen inactivation is not shared with the other purified FDH complex encoded by the *yrhED* operon from *B. subtilis 168*. This other FDH shows a much lower tolerance to oxygen. This is despite the fact that YrhED performs the same formate oxidizing activity and share properties such as pH optima (=9) and a similar level of catalytic efficiency. However, their respective amino acid sequences have only 60% of identity.

References

Arias-Cartin, R., Dobihal, G.S., Campos, M., Surovtsev, I.V., Parry, B., and Jacobs-Wagner, C. (2017). Replication fork passage drives asymmetric dynamics of a critical nucleoid-associated protein in Caulobacter. EMBO J 36, 301-318.

Axley, M.J., Grahame, D.A., and Stadtman, T.C. (1990). Escherichia coli formate-hydrogen lyase. Purification and properties of the selenium-dependent formate dehydrogenase component. J. Biol. Chem. 265, 18213-18218.

Böhmer, N., Hartmann, T., and Leimkühler, S. (2014). The chaperone FdsC for Rhodobacter capsulatus formate dehydrogenase binds the bis-molybdopterin guanine dinucleotide cofactor. FEBS Lett. 588, 531-537.

Dubnau, D., and Davidoff-Abelson, R. (1971). Fate of transforming DNA following uptake by competent Bacillus subtilis. I. Formation and properties of the donor-recipient complex. J. Mol. Biol. 56, 209-221

Glaser, P., Danchin, A., Kunst, F., Zuber, P., and Nakano, M.M. (1995). Identification and isolation of a gene required for nitrate assimilation and anaerobic growth of Bacillus subtilis. Journal of Bacteriology 177, 1112-1115.

Hartmann, T., and Leimkühler, S. (2013). The oxygen-tolerant and NAD+-dependent formate dehydrogenase from Rhodobacter capsulatus is able to catalyze the reduction of CO2 to formate. The FEBS Journal 280, 6083-6096.

Magalon, A., and Mendel, R.R. (2015). Biosynthesis and Insertion of the Molybdenum Cofactor. EcoSal Plus 6.

Maia, L.B., Moura, I., and Moura, J.J.G. (2017). Molybdenum and tungsten-containing formate dehydrogenases: Aiming to inspire a catalyst for carbon dioxide utilization. Inorganica Chimica Acta 455, 350-363.

Nakano, M.M., Dailly, Y.P., Zuber, P., and Clark, D.P. (1997). Characterization of anaerobic fermentative growth of Bacillus subtilis: identification of fermentation end products and genes required for growth. J. Bacteriol. 179, 6749-6755.

Nicolas, P., Mäder, U., Dervyn, E., Rochat, T., Leduc, A., Pigeonneau, N., Bidnenko, E., Marchadier, E., Hoebeke, M., Aymerich, S., et al. (2012). Condition-Dependent Transcriptome Reveals High-Level Regulatory Architecture in Bacillus subtilis. Science 335, 1103-1106.

Nielsen, C.F., Lange, L., and Meyer, A.S. (2019). Classification and enzyme kinetics of formate dehydrogenases for biomanufacturing via CO2 utilization. Biotechnology Advances 37, 107408.

Niks, D., and Hille, R. (2019). Molybdenum- and tungsten-containing formate dehydrogenases and formylmethanofuran dehydrogenases: Structure, mechanism, and cofactor insertion. Protein Science 28, 111-122.

Niks, D., Duvvuru, J., Escalona, M., and Hille, R. (2016). Spectroscopic and Kinetic Properties of the Molybdenum-containing, NAD+-dependent Formate Dehydrogenase from Ralstonia eutropha. J. Biol. Chem. 291, 1162-1174.

Rivers, S.L., McNairn, E., Blasco, F., Giordano, G., and Boxer, D.H. (1993). Molecular genetic analysis of the moa operon of Escherichia coli K-12 required for molybdenum cofactor biosynthesis. Mol. Microbiol. 8, 1071-1081.

Schuchmann, K., and Müller, V. (2013). Direct and Reversible Hydrogenation of CO2 to Formate by a Bacterial Carbon Dioxide Reductase. Science 342, 1382-1385.

Thomé, R., Gust, A., Toci, R., Mendel, R., Bittner, F., Magalon, A., and Walburger, A. (2012). A Sulfurtransferase Is Essential for Activity of Formate Dehydrogenases in Escherichia coli. J. Biol. Chem. 287, 4671-4678.

Zhu, B., and Stülke, J. (2018). SubtiWiki in 2018: from genes and proteins to functional network annotation of the model organism Bacillus subtilis. Nucleic Acids Res 46, D743-D748.

Zuchan, K., Baymann, F., Baffert, C., Brugna, M., and Nitschke, W. (2021). The dyad of the Y-junction- and a flavin module unites diverse redox enzymes. Biochim Biophys Acta Bioenerg 1862, 148401.

**Claims**

1. A YjgC-type protein, said protein comprising an amino acid sequence as set forth in SEQ ID NO: 1 or any homologous sequence derived from said sequence SEQ ID NO:1 by substitution, addition or deletion of at least one amino acid, provided that said homologous sequence retains at least 70 %, preferably at least 99 % of identity with the amino acid sequence set forth in SEQ ID NO:1.

2. The YjgC-type protein as claimed in Claim 1, wherein the amino acid in corresponding to position 392 of a FDH consensus sequence is not an histidine and is advantageously a glutamine.

3. The YjgC-type protein as claimed in Claim 1 or 2, wherein said YjgC-type protein retains an enzymatic activity of formate dehydrogenase and/or of carbon dioxide reductase.

4. The YjgC-type protein as claimed in any one of Claims 1 to 3, wherein said YjgC-type protein originates from a microorganism belonging to the *Bacillales* order, and more particularly a microorganism being a *Bacillus subtilis.*

5. A YjgD-type protein, said protein comprising an amino acid sequence as set forth in SEQ ID NO: 3 or any homologous sequence derived from said sequence SEQ ID NO:3 by substitution, addition or deletion of at least one amino acid, provided that said homologous sequence retains at least 70 %, preferably at least 99 % of identity with the amino acid sequence set forth in SEQ ID NO:3.

6. The YjgD-type protein as claimed in Claim 5, wherein said YjgD-type protein retains an activity of formate dehydrogenase and/or of carbon dioxide reductase, when associated with a YjgC-type protein.

7. The YjgD-type protein as claimed in Claim 5 or 6, wherein said YjgD-type protein originates from a microorganism belonging to the *Bacillales* order, and more particularly a microorganism being a *Bacillus subtilis.*

8. An YjgCD-type protein complex, wherein said complex comprises at least an YjgC-type protein as claimed in any one of Claim 1 to 4 and an YjgD-type protein as claimed in any one of Claim 5 to 7.

9. The YjgCD-type protein complex as claimed Claim 8, wherein said complex has an enzymatic activity of formate dehydrogenase and/or of carbon dioxide reductase.

10. The YjgCD-type protein complex as claimed Claim 8 or 9, wherein said complex is resistant to oxygen exposure and in particular has can retain up to 80% of its activity after 14 hours of atmospheric oxygen exposure

11. A recombinant vector comprising a gene encoding for an YjgC-type protein as claimed in any one of Claim1 to 4, an YjgD-type protein as claimed in any one of Claims 5 to 7, and/or an YjgCD-type protein complex as claimed in Claim 8 or 9.

12. A recombinant microorganism comprising a gene encoding for an YjgC-type protein as claimed in any one of Claim1 to 4, an YjgD-type protein as claimed in any one of Claims 5 to 7, and/or an YjgCD-type protein complex as claimed in Claim 8 or 9.

13. Use of a YjgC-type protein as claimed in any one of Claims 1 to 4, a YjgD-type protein as claimed in any one of Claims 5 to 7, a YjgCD-type protein complex as claimed in any one of Claims 8 to 11, and/or a recombinant microorganism as claimed in Claim 12 to carry out the oxidation of formate to $CO_2$ and/or the reduction of $CO_2$ to formate.

14. A method for producing formic acid and/or fixing carbon dioxide, said method comprising the steps of contacting a medium comprising a YjgC-type protein as claimed in any one of Claims 1 to 4, a YjgD-type protein as claimed in any one of Claims 5 to 7, a YjgCD-type protein complex as claimed in Claim 8 or 9, and/or a recombinant microorganism as claimed in Claim 11 and carbon dioxide.

15. The method of Claim 14, wherein the pH of said medium ranges from 9.5 to 10.5.

**Figure 1**

A.

Mo-BisPGD

4Fe4S

4Fe4S

4Fe4S

4Fe4S    2Fe2S

B.

| | | | |
|---|---|---|---|
| FmdB_METTF | C | RGHC | SEQ ID NO : 9 |
| FmdB_METTM | C | RGHC | SEQ ID NO : 9 |
| FmdB_METWO | C | RGHC | SEQ ID NO : 9 |
| FmdB_METBA | C | RGHC | SEQ ID NO : 9 |
| FwdB_METKA | C | RGHY | SEQ ID NO : 10 |
| FwdB_METTF | C | RGHY | SEQ ID NO : 10 |
| FwdB_METWO | C | RGHY | SEQ ID NO : 10 |
| FwdB_ARCFU | C | RGHY | SEQ ID NO : 10 |
| FdhF_CORGL | C | RGHS | SEQ ID NO : 11 |
| FdhA_WOLSU | C | RGHD | SEQ ID NO : 12 |
| FdhA_SULMU | U | RGHD | SEQ ID NO : 12 |
| FdnG_DESVH | U | RGEP | SEQ ID NO : 13 |
| FdhA_DESDE | U | RGEP | SEQ ID NO : 13 |
| FdhA_DESGI | U | RGEA | SEQ ID NO : 14 |
| FdhA_DESAG | U | RGES | SEQ ID NO : 15 |
| FdnG_PSEAE | U | RGHS | SEQ ID NO : 16 |
| FdoG_ECOLI | U | RGHS | SEQ ID NO : 16 |
| Fdh_CITS77 | U | RGHS | SEQ ID NO : 16 |
| FdnG_ECOLI | U | RGHS | SEQ ID NO : 16 |
| YjgC_BACSU | C Q | RGHN | SEQ ID NO : 17 |
| FdsA_CUPNE | C | RGQN | SEQ ID NO : 18 |
| Fdh_RHOCB | C | RGQN | SEQ ID NO : 18 |
| Fdh_METTR | C | RGQN | SEQ ID NO : 18 |
| FdsA_RHOPA | C | RGQN | SEQ ID NO : 18 |
| Fdh1A_METEX | C | RGQN | SEQ ID NO : 18 |
| Fdh_SYNFM | C | RGQN | SEQ ID NO : 18 |
| Fdh_MOOTH | U | RGQS | SEQ ID NO : 19 |
| FdhAI_PEPAC | U | RGQN | SEQ ID NO : 18 |
| FdhAII_PEPAC | U | RGQN | SEQ ID NO : 18 |
| FdhF2_GOTA9 | U | RGQN | SEQ ID NO : 18 |
| FdhA_METFO | C | RGQN | SEQ ID NO : 18 |
| FdhA_METTF | C | RGQN | SEQ ID NO : 18 |
| FdhA2_METMI | U | RGQN | SEQ ID NO : 18 |
| FdhA1_METMI | U | RGQN | SEQ ID NO : 18 |
| Fdh_METVS | U | RGQN | SEQ ID NO : 18 |
| FdhF_THEKI | C | RGQN | SEQ ID NO : 18 |
| FdhF_ECOLI | U | RGQN | SEQ ID NO : 18 |
| FdhF1_ACEWD | C | RGQN | SEQ ID NO : 18 |
| FdhF2_ACEWD | U | RGQN | SEQ ID NO : 18 |
| FdhF_CLOPA | C | RGQN | SEQ ID NO : 18 |
| FdhF_9CLOT | C | RGQN | SEQ ID NO : 18 |
| FdhF_CLOLD | C | RGQN | SEQ ID NO : 18 |

Sequence similarity % ≥100 ≥80

C.

yjgA yjgB   yjgC   yjgD

1284370            128990

**Figure 2**

Figure 3

Figure 4

## SEQ ID NO : 1

```
MAGKKTITIN GVEMEASEEQ TVLQLLNNSS IEVPQVCYHP SLGPIETCDT CIVSINGELK
RSCSAELKDG DVIDTLSPDV KKAQVIGMDK ILYNHELYCT VCDYNNGGCE IHNTVKEMKI
NHQSIPFDHK PYHKDESHPF YRYDPDQCIL CGRCVEACQD VQVTETLTID WERKRPRVIW
DNDVPINESS CVSCGHCSTV CPCNAMMEKG MEGEAGYLTG INNETLRPMI EITKGVETGY
GSILAISDME SAMRDERIKK TKTVCTYCGV GCSFDVWTKG RDILKVEPQE EAPANGISTC
VKGKFGWDFV NSEERLTKPL IREGDHFREA EWEEALLLIA SKFTELKEAF GPDSLAFITS
SKCTNEESYL MQKLARGVIG TNNVDNCSRY CQSPATAGLF RTVGYGGDSG SITDIAQADL
VLIIGSNTSE SHPVLSTRIK RAHKLRGQKV IVADIRKHEM AERSDLFVQP RAGSDIVWLN
AIAKYLIENG KADERFLRER VNGRDEYVKS LAPYTLEYAE EKTGIDQETL IQMAEMIGQA
DSVCALWAMG VTQHIGGSDT STAISNLLLV TGNYGKPGAG SYPLRGHNNV QGASDFGSMP
DRLPGYEKVT DEQVRQKYER VWGVPLPKEP GMTNHEMIEK IHSGQLKAMY VKGEEMGLVD
SNINHVHAAY EKLDFFVVQD IFLSRTAEFA DVVLPASPSL EKEGTFTNTE RRIQRLYQVF
EPLGESKPDW QIIMEVANKL GAGWLYEHPA DIMEEAAKLS PIYAGVTYER LEGYNSLQWP
VNADGKDSPL LFTERFPFPD GKAILYPVQW TEPKEFGEEY DIHVNNGRLL EHFHEGNLTY
KSKGISEKTP EVFLEISPEL AAERGIQDGT LVRLTSPFGN VKVKCLITDR VKGKEVYLPM
NDSGEAAINL LTGSHADKDT DTPAYKETSA KMEILKHDGI SPLPKINHRN GNPQPQIGVQ
VHKKWARKDY IFPGDAVKRG MGHNG
```

Figure 5

**SEQ ID NO : 2**

```
ATGGCTGGCA AGAAAACAAT CACAATAAAC GGCGTTGAAA TGGAAGCGTC CGAGGAACAG
ACGGTGCTGC AGCTTTTGAA TAACAGCTCG ATTGAAGTGC CGCAAGTTTG CTATCATCCA
AGCTTAGGGC CGATTGAGAC ATGTGATACG TGCATCGTCA GCATCAATGG TGAGCTGAAG
AGGTCGTGCT CCGCTGAATT AAAGGATGGG GATGTCATTG ATACGCTCTC ACCTGACGTG
AAAAAAGCGC AGGTCATCGG GATGGACAAG ATTTTATATA ATCATGAGCT TTATTGTACA
GTTTGCGATT ACAACAATGG GGGATGCGAA ATACATAATA CGGTGAAGGA AATGAAAATC
AATCACCAAA GCATTCCGTT TGATCACAAG CCATACCATA AGGATGAATC CCATCCGTTT
TATCGGTATG ATCCAGATCA ATGTATATTG TGCGGACGCT GTGTTGAGGC ATGCCAAGAC
GTTCAGGTAA CTGAAACGCT GACCATTGAC TGGGAGCGGA AACGCCCGCG CGTCATTTGG
GATAACGACG TGCCGATCAA TGAGTCGTCA TGTGTGTCAT GCGGCCATTG TTCAACGGTC
TGTCCGTGCA ACGCGATGAT GGAAAAAGGA ATGGAAGGGG AAGCCGGATA TTTAACAGGC
ATCAATAACG AAACGCTGCG ACCGATGATC GAGATCACGA AAGGCGTAGA GACAGGCTAC
GGCTCGATTC TCGCGATTTC TGATATGGAA TCGGCCATGC GTGATGAACG AATCAAAAAA
ACGAAAACCG TCTGCACGTA TTGCGGCGTC GGCTGCAGCT TTGATGTCTG GACAAAGGGC
AGAGACATTC TGAAAGTAGA GCCGCAGGAG GAAGCGCCTG CCAACGGCAT TTCTACTTGT
GTGAAAGGCA AGTTCGGCTG GGATTTCGTT AACAGCGAAG AACGCCTGAC AAAGCCGCTG
ATCCGTGAAG GCGATCATTT CCGTGAAGCA GAGTGGGAGG AGGCGTTATT GCTGATCGCC
AGCAAGTTCA CTGAATTAAA AGAGGCGTTT GGCCCGGATT CTCTCGCTTT TATTACATCT
TCTAAATGTA CAAATGAAGA ATCCTATCTT ATGCAAAGC TGGCCAGAGG GGTCATTGGC
ACAAATAATG TGGACAACTG CTCTCGCTAT TGCCAATCTC CTGCGACTGC CGGCCTGTTC
CGGACAGTCG GCTATGGCGG TGATTCAGGA TCAATTACAG ATATCGCGCA AGCGGACCTT
GTTTTGATTA TCGGTTCGAA CACGTCTGAG TCGCATCCAG TCTTATCCAC TCGGATTAAA
CGGGCTCATA AGCTGAGAGG GCAGAAAGTG ATCGTCGCGG ATATCAGAAA GCACGAAATG
GCAGAGCGCT CAGATTTATT TGTCCAGCCG CGTGCCGGAT CGGATATCGT CTGGCTGAAT
GCGATCGCCA AATATTTAAT AGAAAACGGC AAGGCCGATG AACGATTTTT GAGAGAAAGA
GTGAACGGAC GGGATGAATA TGTAAAAAGC CTCGCGCCGT ACACACTTGA ATATGCTGAG
GAGAAAACCG GGATTGATCA AGAAACCCTT ATCCAAATGG CAGAGATGAT CGGGCAAGCT
GACAGTGTGT GCGCATTGTG GGCGATGGGC GTGACACAGC ATATTGGAGG AAGCGACACG
AGCACGGCGA TCTCTAATCT GCTGCTTGTG ACAGGAAACT ACGGAAAACC GGGAGCAGGT
TCTTACCCAT TGCGCGGCCA TAACAACGTG CAGGGAGCAA GTGACTTTGG GAGTATGCCT
GACAGACTAC CGGGCTATGA AAAAGTGACG GACGAACAAG TCCGCCAAAA ATACGAGCGT
GTGTGGGGTG TGCCGCTGCC GAAGGAGCCG GGTATGACCA ACCACGAAAT GATTGAAAAA
ATCCATTCCG GACAGTTGAA AGCGATGTAT GTAAAAGGTG AAGAAATGGG CCTTGTCGAC
TCCAATATCA ATCATGTACA CGCTGCATAT GAAAAGCTTG ATTTCTTTGT GGTGCAGGAC
ATTTTCCTTT CACGTACAGC GGAGTTTGCT GATGTGGTTC TTCCTGCGAG CCCGAGCCTT
GAAAAGAAG GAACGTTTAC AAACACAGAA CGCCGGATTC AGCGTTTGTA TCAGGTATTT
GAACCGCTGG GTGAATCAAA GCCGGATTGG CAGATTATTA TGGAGGTTGC CAATAAGCTT
GGCGCCGGCT GGCTCTATGA GCATCCTGCA GACATTATGG AGGAAGCAGC CAAGCTGTCG
CCGATTTATG CCGGTGTCAC CTATGAACGT CTTGAGGGCT ATAATTCCCT GCAATGGCCT
GTAAACGCTG ATGGAAAGGA TTCGCCTTTA CTCTTTACAG AGCGCTTCCC TTTCCCAGAC
GGCAAGGCCA TTCTCTACCC TGTCCAGTGG ACAGAGCCTA AGGAATTCGG TGAGGAATAT
GATATCCATG TCAACAATGG GCGGCTCTTG GAGCACTTCC ACGAAGGGAA CCTGACTTAC
AAATCAAAAG GGATTTCGGA GAAAACACCG GAAGTGTTCT TAGAAATTTC TCCTGAGCTT
GCGGCAGAAC GAGGGATTCA GGACGGAACC CTCGTCAGAC TCACATCGCC TTTCGGAAAT
GTCAAAGTGA AGTGCCTGAT TACTGATCGA GTCAAAGGA AGGAAGTATA TCTGCCAATG
AACGATTCAG GGGAAGCGGC GATCAACCTG TTAACAGGCA GCCATGCCGA TAAGGATACC
GATACGCCTG CTTACAAAGA AACATCGGCC AAGATGGAAA TTTTGAAGCA CGACGGAATC
AGCCCGCTGC CTAAGATCAA CCACCGCAAC GGCAATCCGC AGCCGCAAAT CGGTGTGCAG
GTTCATAAGA AATGGGCGCG GAAGGATTAT ATTTTCCCTG TGACGCTGT GAAAAGGGGG
ATGGGCCATA ATGGCTAA
```

**Figure 6**

**SEQ ID NO : 3**

```
MAKAIKRIQK IEVTEEDQRK RDLREIEDAL IDHKEAILET LHMLGHMNER GVLPLLRGLF
GQGDKVLDIL VKKADTEETA NTLKNLLLLF GTLGMLDVKQ LEPLILKVNA GVASAVEQKN
SEEKTGYFDI IRSLKDPEIN KSITLLFSFL KGMGQDTKEL ERTTQPPEHQ KHHQEPREKR
GMNKRD
```

**Figure 7**

**SEQ ID NO : 4**

```
ATGGCTAAAG CGATTAAACG AATCCAAAAA ATCGAGGTAA CAGAAGAGGA TCAGCGAAAG
CGTGATTTGC GGGAAATTGA AGATGCTCTA ATTGACCACA AGAAGCGAT TCTCGAAACA
TTGCATATGC TGGGCCATAT GAACGAGCGC GGGGTCCTGC CGTTGCTCCG CGGCCTTTTT
GGTCAAGGGG ATAAAGTTCT TGATATCCTG GTGAAAAAGG CAGATACAGA GGAAACAGCC
AATACGCTGA AAAATCTGCT TCTTCTGTTT GGAACACTCG GCATGCTGGA CGTGAAGCAG
CTGGAGCCTC TCATTTTGAA AGTGAATGCG GGTGTGGCAA GCGCTGTAGA GCAGAAAAAC
AGTGAAGAAA AGACAGGTTA TTTTGATATT ATCCGCTCGC TAAAAGACCC GGAAATCAAC
AAATCAATTA CACTGCTCTT TTCCTTTTTA AAAGGAATGG GACAAGATAC AAAAGGAGCTG
GAACGCACGA CGCAGCCTCC GGAACACCAA AAACATCATC AGGAACCCCG AGAGAAAAGA
GGAATGAATA AGCGAGATTG A
```

**Figure 8**

Europäisches
Patentamt

European
Patent Office

Office européen
des brevets

## EUROPEAN SEARCH REPORT

**Application Number**

EP 21 30 5965

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RIVOLTA C ET AL: "A 35-7 KB DNA FRAGMENT FROM THE BACILLUS SUBTILIS CHROMOSOME CONTAINING A PUTATIVE 12-3 KB OPERON INVOLVED IN HEXURONATE CATABOLISM AND A PERFECTLY SYMMETRICAL HYPOTHETICAL CATABOLITE-RESPONSIVE ELEMENT", MICROBIOLOGY, SOCIETY FOR GENERAL MICROBIOLOGY, READING, vol. 144, 1 January 1998 (1998-01-01), pages 877-884, XP002928242, ISSN: 1350-0872 * the whole document * -& DATABASE UniProt [Online] 28 July 2009 (2009-07-28), "Probable oxidoreductase YjgC", XP002804957, Database accession no. O34720 * sequence information; the whole document * -& DATABASE UniProt [Online] 3 November 2009 (2009-11-03), "Uncharacterized protein YjgD", XP002804958, Database accession no. O34681 * sequence information; the whole document * ----- | 1-7,11, 12 | INV. C12N9/02 |
| X | US 2003/233675 A1 (CAO YONGWEI [US] ET AL) 18 December 2003 (2003-12-18) * sequence 17701 * ----- | 1-4,11, 12 | |

TECHNICAL FIELDS SEARCHED (IPC)

C12N

-/--

The present search report has been drawn up for all claims

| Place of search | Date of completion of the search | Examiner |
|---|---|---|
| Munich | 7 December 2021 | Bladier, Cecile |

EPO FORM 1503 03.82 (P04C01)

page 1 of 3

# EUROPEAN SEARCH REPORT

**Application Number**

**EP 21 30 5965**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | J. MOSTERTZ: "Transcriptome and proteome analysis of Bacillus subtilis gene expression in response to superoxide and peroxide stress", MICROBIOLOGY, vol. 150, no. 2, 1 February 2004 (2004-02-01), pages 497-512, XP055222730, Reading ISSN: 1350-0872, DOI: 10.1099/mic.0.26665-0 * page 498, right-hand column, lines 10-13, paragraph 2 * * table 1 * * page 507, right-hand column, paragraph 2 * | 8-10 | |
| A | NIELSEN CHRISTIAN FØRGAARD ET AL: "Classification and enzyme kinetics of formate dehydrogenases for biomanufacturing via CO2 utilization", BIOTECHNOLOGY ADVANCES, ELSEVIER PUBLISHING, BARKING, GB, vol. 37, no. 7, 12 June 2019 (2019-06-12), XP085893521, ISSN: 0734-9750, DOI: 10.1016/J.BIOTECHADV.2019.06.007 [retrieved on 2019-06-12] * page 8, right-hand column, paragraph 3 * | 1-15 | |

TECHNICAL FIELDS SEARCHED (IPC)

-/--

The present search report has been drawn up for all claims

| Place of search | Date of completion of the search | Examiner |
|---|---|---|
| Munich | 7 December 2021 | Bladier, Cecile |

EPO FORM 1503 03.82 (P04C01)

**page 2 of 3**

Europäisches Patentamt

European Patent Office

Office européen des brevets

# EUROPEAN SEARCH REPORT

Application Number

EP 21 30 5965

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PETERSOHN ANJA ET AL: "Global Analysis of the General Stress Response of Bacillus subtilis", JOURNAL OF BACTERIOLOGY (PRINT), vol. 183, no. 19, 1 October 2001 (2001-10-01), pages 5617-5631, XP55868733, US ISSN: 0021-9193, DOI: 10.1128/JB.183.19.5617-5631.2001 * table 1 * | 1-15 | |
| A,D | AXLEY M J ET AL: "Escherichia coli formate-hydrogen lyase. Purification and properties of the selenium-dependent formate dehydrogenase component", JOURNAL OF BIOLOGICAL CHEMISTRY, AMERICAN SOCIETY FOR BIOCHEMISTRY AND MOLECULAR BIOLOGY, US, vol. 265, no. 30, 25 October 1990 (1990-10-25), pages 18213-18218, XP002999332, ISSN: 0021-9258 * page 18217, right-hand column, paragraph 5 – page 18218, left-hand column, paragraph 1; figure 7 * | 1-15 | TECHNICAL FIELDS SEARCHED (IPC) |

The present search report has been drawn up for all claims

| Place of search | Date of completion of the search | Examiner |
|---|---|---|
| Munich | 7 December 2021 | Bladier, Cecile |

EPO FORM 1503 03.82 (P04C01)

## ANNEX TO THE EUROPEAN SEARCH REPORT
## ON EUROPEAN PATENT APPLICATION NO.

EP 21 30 5965

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2021

| Patent document cited in search report | | Publication date | Patent family member(s) | | Publication date |
|---|---|---|---|---|---|
| US 2003233675 | A1 | 18-12-2003 | US 2003233675 | A1 | 18-12-2003 |
| | | | US 2008229451 | A1 | 18-09-2008 |
| | | | US 2015299720 | A1 | 22-10-2015 |
| | | | US 2017342433 | A1 | 30-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013011292 A

**Non-patent literature cited in the description**

- **ARIAS-CARTIN, R. ; DOBIHAL, G.S. ; CAMPOS, M. ; SUROVTSEV, I.V. ; PARRY, B. ; JACOBS-WAGNER, C.** Replication fork passage drives asymmetric dynamics of a critical nucleoid-associated protein in Caulobacter. *EMBO J,* 2017, vol. 36, 301-318
- **AXLEY, M.J. ; GRAHAME, D.A. ; STADTMAN, T.C.** Escherichia coli formate-hydrogen lyase. Purification and properties of the selenium-dependent formate dehydrogenase component. *J. Biol. Chem.,* 1990, vol. 265, 18213-18218
- **BÖHMER, N. ; HARTMANN, T. ; LEIMKÜHLER, S.** The chaperone FdsC for Rhodobacter capsulatus formate dehydrogenase binds the bis-molybdopterin guanine dinucleotide cofactor. *FEBS Lett,* 2014, vol. 588, 531-537
- **DUBNAU, D. ; DAVIDOFF-ABELSON, R.** Fate of transforming DNA following uptake by competent Bacillus subtilis. I. Formation and properties of the donor-recipient complex. *J. Mol. Biol.,* 1971, vol. 56, 209-221
- **GLASER, P. ; DANCHIN, A. ; KUNST, F. ; ZUBER, P. ; NAKANO, M.M.** Identification and isolation of a gene required for nitrate assimilation and anaerobic growth of Bacillus subtilis. *Journal of Bacteriology,* 1995, vol. 177, 1112-1115
- **HARTMANN, T. ; LEIMKÜHLER, S.** The oxygen-tolerant and NAD+-dependent formate dehydrogenase from Rhodobacter capsulatus is able to catalyze the reduction of CO2 to formate. *The FEBS Journal,* 2013, vol. 280, 6083-6096
- **MAGALON, A. ; MENDEL, R.R.** Biosynthesis and Insertion of the Molybdenum Cofactor. *EcoSal Plus,* 2015, vol. 6
- **MAIA, L.B. ; MOURA, I. ; MOURA, J.J.G.** Molybdenum and tungsten-containing formate dehydrogenases: Aiming to inspire a catalyst for carbon dioxide utilization. *Inorganica Chimica Acta,* 2017, vol. 455, 350-363
- **NAKANO, M.M. ; DAILLY, Y.P. ; ZUBER, P. ; CLARK, D.P.** Characterization of anaerobic fermentative growth of Bacillus subtilis: identification of fermentation end products and genes required for growth. *J. Bacteriol.,* 1997, vol. 179, 6749-6755
- **NICOLAS, P. ; MÄDER, U. ; DERVYN, E. ; ROCHAT, T. ; LEDUC, A. ; PIGEONNEAU, N. ; BIDNENKO, E. ; MARCHADIER, E. ; HOEBEKE, M. ; AYMERICH, S. et al.** Condition-Dependent Transcriptome Reveals High-Level Regulatory Architecture in Bacillus subtilis. *Science,* 2012, vol. 335, 1103-1106
- **NIELSEN, C.F. ; LANGE, L. ; MEYER, A.S.** Classification and enzyme kinetics of formate dehydrogenases for biomanufacturing via CO2 utilization. *Biotechnology Advances,* 2019, vol. 37, 107408
- **NIKS, D. ; HILLE, R.** Molybdenum- and tungsten-containing formate dehydrogenases and formylmethanofuran dehydrogenases: Structure, mechanism, and cofactor insertion. *Protein Science,* 2019, vol. 28, 111-122
- **NIKS, D. ; DUVVURU, J. ; ESCALONA, M. ; HILLE, R.** Spectroscopic and Kinetic Properties of the Molybdenum-containing, NAD+-dependent Formate Dehydrogenase from Ralstonia eutropha. *J. Biol. Chem.,* 2016, vol. 291, 1162-1174
- **RIVERS, S.L. ; MCNAIRN, E. ; BLASCO, F. ; GIORDANO, G. ; BOXER, D.H.** Molecular genetic analysis of the moa operon of Escherichia coli K-12 required for molybdenum cofactor biosynthesis. *Mol. Microbiol.,* 1993, vol. 8, 1071-1081
- **SCHUCHMANN, K. ; MÜLLER, V.** Direct and Reversible Hydrogenation of CO2 to Formate by a Bacterial Carbon Dioxide Reductase. *Science,* 2013, vol. 342, 1382-1385
- **THOMÉ, R. ; GUST, A. ; TOCI, R. ; MENDEL, R. ; BITTNER, F. ; MAGALON, A. ; WALBURGER, A.** A Sulfurtransferase Is Essential for Activity of Formate Dehydrogenases in Escherichia coli. *J. Biol. Chem.,* 2012, vol. 287, 4671-4678

- **ZHU, B. ; STÜLKE, J.** SubtiWiki in 2018: from genes and proteins to functional network annotation of the model organism Bacillus subtilis. *Nucleic Acids Res,* 2018, vol. 46, D743-D748

- **ZUCHAN, K. ; BAYMANN, F. ; BAFFERT, C. ; BRUGNA, M. ; NITSCHKE, W.** The dyad of the Y-junction- and a flavin module unites diverse redox enzymes. *Biochim Biophys Acta Bioenerg,* 2021, vol. 1862, 148401